# EUROPEAN PATENT APPLICATION

(11) **EP 1 258 240 A1**
(43) Date of publication of application: **20.11.2002**
(21) Application number: 01112217.3
(22) Date of filing: 18.05.2001
(51) Int. Cl.: A61K 7/48, A41B 9/00, D06M 13/00

(54) **Device for the controlled release of vegetable extracts in contact with the human skin**

(71) Applicant: Gizeta Calze Srl, 46042 Castel Goffredo (MN) (IT)
(72) Inventor: Frizzi, Giancarlo, 46042 Castel Goffredo (MN) (IT)
(74) Representative: Frignoli, Luigi

(57) **Abstract**

A device for the controlled release of vegetable extracts in contact with the human skin, consisting of a fabric on which the vegetable extracts are firmly secured and resist repeated washes.

## Description

The present invention relates to a device for the controlled release of vegetable extracts in contact with the human skin.

The vegetable extracts are in the form of essential oils in the dry or fluid state.

Many vegetable extracts are commonly used for treating the human skin, for cosmetic and sometimes pharmacological purposes.

The present invention relates to those vegetable extracts which are accepted by international Pharmacopeas.

Extracts of ginseng, ginkgo biloba, centella asiatica, geranium, aloe, camomile, equisetum etc. can be cited as examples.

These vegetable extracts are commonly used in the form of creams, lotions or the like, which are spread onto the human skin to enable these extracts to exhibit their cosmetic or pharmacological activity: this form of administration has the drawback that the duration of their contact with the skin is very short.

An attempt was made to prolong the time in which the vegetable extracts remain in contact with the skin by impregnating a fabric or similar article with these extracts (to then be used by maintaining it applied to the skin): however, with this method the administration of the vegetable extracts is not always improved in practice.

As liposomes are innocuous to the human health and are known vegetable extract carriers, it was attempted to mix phospholipids (liposome precursors) with vegetable extracts to obtain a homogeneous fluid mass with which the fabric articles were impregnated, however these proved incapable of retaining significant quantities of such extracts.

It has been surprisingly found that if the vegetable extracts are homogeneously mixed with biomimetic phospholipids, a homogeneous mass is obtained with which a fabric article can be impregnated to the extent that it retains sufficient quantities of vegetable extracts to significantly develop their typical activity when in contact with the human skin, and gradually release these extracts onto the skin with time; this signifies that the behaviour of biomimetic phospholipids is totally different from that of common phospholipids.

Biomimetic phospholipids are well known in the literature and are described for example by:
D.L. Fost. Multifunctional biomimetic phospholipids: natural base ingredients for personal care products. Asian COSFA '95 Convention Proceedings, Bangkok, Thailand, 1995;
J.I. Yablonski and D.L. Fost. Synthetic phospholipids: a new class of multifunctional preservative/surfactants. In: Cosmetics Proceedings, Reed Exhibitions, Richmond, United Kingdom, 1991;
J.I. Yablonski and D.L. Fost. Synthetic phospholipid-II - Multifunctional preservative/surfactants - an update. Presented at the IVth International Meeting on Cosmetic Dermatology, Rome, November 1991; and
J.J. Kabara. Preservative-free and self-preserving cosmetics and drugs. Edited by Marcel Dekker Inc., New York, Chapter 6, pages 139- 157, 1997.

To obtain the devices of the invention, between 98.6% and 99.99% of fresh water, between 0.001% and 0.5% of vegetable extracts and between 0.0099% and 0.9% of biomimetic phospholipids, the percentages being by weight, are fed into a mixer, agitation is applied until a homogeneous liquid mass is obtained while maintaining the temperature between 25°C and 35°C, fabric articles are immersed into this homogeneous mass and a fabric softener is added in a quantity of between 0.5% and 1% on the weight of the homogeneous mass, the liquid mass is maintained at the aforesaid temperature and is circulated in contact with the fabric articles for at least 15 minutes, the fabric articles are removed and then centrifuged such that they contain a residue of liquid mass less than about 10% on the weight of the articles, and the articles are dried such that they contain less than 5% of this mass on the total weight of the fabric article, on which the vegetable extracts are found to be securely fixed and gradually releasable when in contact with the human skin.

In particular, to obtain said homogeneous liquid mass the mixture of its components is passed through a heat exchanger heated to a temperature between 100°C and 120°C, the mixture remaining in this heat exchanger for a time between 1 second and 10 seconds and being then immediately cooled to a temperature between 25°C and 35°C.

The devices according to the invention are characterised in that said vegetable extracts are in the form of complexes in which they are bonded to biomimetic phospholipids, these complexes at least partly covering the textile fibres of a fabric formed from the fibres.

In particular said complexes are present in a quantity between 0.001% and 0.7% on the total weight of the device.

Preferably in said complexes the percentage weight ratios of the vegetable extracts to the biomimetic phospholipids lie between 1:1 and 1:10.

To further clarify the understanding of the nature and characteristics of the devices for the controlled release of vegetable extracts in contact with the human skin and their method of preparation, some non- limiting examples will now be described.

### EXAMPLE 1

### Preparation of the homogeneous mass

450 g of fresh water maintained at a temperature between 25 and 35°C, 125 g of stearamidopropyl phosphatidyl PG-dimonium chloride (biomimetic phospholipid), 25 g of dry extract of Ginkgo Biloba, 25 g of dry extract of Ginseng and 0.3 g of essential oil of melissa are fed into a mixer of 500 litre capacity, and agitation applied for 10 minutes to obtain a homogeneous mass.

### Preparation of the article

50 kg of polyamide fibre fabric stockings are immersed in the aforesaid homogeneous mass, 2.5 kg of a silicone softener are added and the homogeneous mass circulated for 20-25 minutes.

After this period the polyamide fabric articles are removed and centrifuged to eliminate most of the liquid, after which they are dried by a steam-heated plate.

The stockings obtained in this manner have a liquid content less than 3%.

The stockings were subjected to automaton tolerability tests and were well tolerated. Tests were carried out to evaluate the residence time of the extracts on the stockings and in particular their resistance to washing.

After three washes there was still a significant presence of vegetable extracts.

### EXAMPLE 2

### Preparation of the homogeneous mass

95 litres of fresh water maintained at a temperature between 25 and 35°C, 10 g of cocamidopropyl PG-dimonium chloride phosphate, 1 g of essential oil of coriander and 1 g of essential oil of eucalyptus are fed into a mixer of 100 litre capacity, and agitation applied for 10 minutes to obtain a homogeneous mass.

### Preparation of the article

8 litres of a natural fibre fabric article are immersed in the previously prepared homogeneous mass, 250 g of a cationic softener are added, and the homogeneous mass is circulated for 20 minutes. After this period the natural fibre fabric articles are removed, centrifuged and then further dried by steam-heated plates.

The underpants obtained in this manner have a liquid content less than 3%.

These articles possess excellent tolerability and develop a refreshing action.

Their action was found to be effective even after the first wash.

### EXAMPLE 3

### Preparation of the homogeneous mass

198 kg of fresh water maintained at a temperature between 25 and 35°C, 50 g of cocamidopropyl PG-dimonium chloride phosphate and 50 g of stearamidopropyl phosphatidyl PG-dimonium chloride (biomimetic phospholipids), 60 g of aloe extract, 20 g of phytosama of centella and 1 g of essential oil of white birch are fed into a mixer of 200 litre capacity.

In order to homogenize this mass it was necessary to pass it through an external heat exchanger in order to heat it for a time less than 5 seconds and rapidly return it to a temperature of 25-35°C.

After this procedure the liquid mass was agitated for 10 minutes to obtain a homogeneous mass.

### Preparation of the article

25 kg of polyamide fibre fabric articles are immersed in the previously prepared homogeneous mass, 100 g of a silicone softener are added and the homogeneous mass circulated for 20-25 minutes. The articles are removed, centrifuged and further dried by heating plates.

The stocking obtained has a liquid content less than 3%.

The articles were subjected to skin tolerability tests and showed a high capacity for hydrating and providing nutriment to the skin.

This activity remains even after the first wash.

## Claims

1. A device for the controlled release of vegetable extracts in contact with the human skin, **characterised in that** said vegetable extracts are in the form of complexes in which they are bonded to biomimetic phospholipids, these complexes at least partly covering the textile fibres of a fabric formed from the fibres.

2. A device as claimed in claim 1, **characterised in that** said complexes are present in a quantity between 0.001% and 0.7% on the total weight of the device.

3. A device as claimed in claim 2, **characterised in that** in said complexes the percentage weight ratios of said vegetable extracts to said biomimetic phospholipids are between 1:1 and 1:10.

4. A method for producing the devices claimed in claims from 1 to 3, **characterised in that** between 98.6% and 99.99% of fresh water, between 0.001% and 0.5% of vegetable extracts and between 0.0099% and 0.9% of biomimetic phospholipids, the percentages being by weight, are fed into a mixer, agitation is applied until a homogeneous liquid mass is obtained while maintaining the temperature between 25°C and 35°C, fabric articles are immersed into this homogeneous mass and a fabric softener is added in a quantity of between 0.5% and 1% on the weight of the homogeneous mass, the liquid mass is maintained at the aforesaid temperature and is circulated in contact with the fabric articles for at least 15 minutes, the fabric articles are removed and then centrifuged such that they contain a residue of liquid mass less than about 10% on the weight of the articles, and the articles are dried such that they contain less than 5% of this mass on the total weight of the fabric article, on which the vegetable extracts are found to be securely fixed and gradually releasable when in contact with the human skin.

5. A method as claimed in claim 4, **characterised in that** to obtain said homogeneous liquid mass the mixture of its components is passed through a heat exchanger heated to a temperature between 100°C and 120°C, the mixture remaining in this heat exchanger for a time between 1 second and 10 seconds and being then immediately cooled to a temperature between 25°C and 35°C.
